Europäisches Patentamt

European Patent Office

Office européen des brevets

(11) Publication number: **0 255 210 B1**

(12) **EUROPEAN PATENT SPECIFICATION**

(45) Date of publication of patent specification: **09.09.92** (51) Int. Cl.⁵: **A61K 7/16**

(21) Application number: **87304789.8**

(22) Date of filing: **29.05.87**

(54) **Oral composition.**

(30) Priority: **27.06.86 JP 152539/86**
             **27.06.86 JP 152540/86**

(43) Date of publication of application:
   **03.02.88 Bulletin  88/05**

(45) Publication of the grant of the patent:
   **09.09.92 Bulletin  92/37**

(84) Designated Contracting States:
   **AT BE CH DE ES FR GB IT LI NL SE**

(56) References cited:
   **JP-A-59 101 418**
   **US-A- 4 051 159**

   **CHEMICAL ABSTRACTS, vol. 105, no. 18, 03
   November 1986, Columbus, OH (US); p. 362,
   no. 158655p**

(73) Proprietor: **LION CORPORATION**
   **3-7, Honjo 1-chome**
   **Sumida-ku Tokyo(JP)**

(72) Inventor: **Tokumitsu, Fumihiko**
   **5-12-4, Kitami**
   **Setagaya-ku Tokyo(JP)**
   Inventor: **Gomi, Tetsuo**
   **3-24-7 Kouyama**
   **Nerima-ku Tokyo(JP)**
   Inventor: **Minemoto, Isao**
   **3-7-2, Kitakashiwa**
   **Kashiwa-shi Chiba(JP)**

(74) Representative: **Adams, William Gordon et al
   RAWORTH, MOSS & COOK 36 Sydenham
   Road
   Croydon Surrey CR0 2EF(GB)**

## Description

The present invention relates to an aqueous oral composition containing a higher aliphatic alcohol having 16 to 28 carbon atoms in a liquid state at an ambient temperature, which can improve the feeling upon application of oral compositions containing silicates by suppressing an astringent taste and chalky taste stemming from the silicate, and which can prevent the escape of flavors from plastic containers by permeation when oral compositions are contained in such plastic containers.

Various abrasives, for example, calcium abrasives such as calcium phosphate (dibasic) and calcium carbonate; aluminum abrasives such as alumina hydrates; and silicate abrasives such as silicic anhydride, alumino silicate, zircono silicate are known as abrasives that can be formulated into oral compositions such as dentifrices. In addition, it is also known in the art that silicates such as silicic acid can be formulated into oral compositions such as dentifrices, as a binder or a brightener.

However, oral compositions such as dentifrices containing silicates leave a particular astringent taste and chalky taste in the mouth, caused by the formulation of silicates into the oral compositions, whereby the feeling upon application thereof is not very good.

Various attempts have been made to solve these problems in oral compositions containing silicates. For example, JP-A-57-171909 (Kokai) proposes the formulation of amino acids and polypeptides for improving the feeling upon application of oral compositions containing silicates,and JP-A-58-118507 (Kokai) proposes the formulation of amides into oral compositions containing silicates. These components can improve the particular astringent taste caused by the formulation of silicates into the oral compositions, but these components also give a particular taste to the compositions, and thus reduce the effect of the desired flavoring of the oral compositions.

Further, a variety of containers for oral compositions have been used, due to marketing demands and, as a result, oral compositions contained in plastic pumping containers and plastic blow tubes have been marketed. However, these plastic containers, especially those made of plastics having an oxygen permeability constant of 1 ml/100 inch$^2 \cdot$24 hr.$\cdot$atm (at room temperature, thickness = 1 mil) or more are disadvantageous, when compared to containers made of metal (e.g., aluminum) or laminated films obtained by laminating plastic films with metal foils (e.g., aluminum foil), in that flavors or perfumes formulated in oral compositions can permeate therethrough and thus escape from the compositions. As a result, the flavoring of oral compositions contained in plastic containers is lost during the storage thereof. Attempts have been made to alleviate these problems by flavoring oral compositions with flavors having a relatively low volatility, such as cinnamic aldehyde, cineol, and methyl salicylate. However, these flavors still do not have sufficient stability, and there is little freedom in the development of a flavor system because flavor materials having relatively high volatility are not stable and, therefore, cannot be used. Thus, the flavoring disadvantageously becomes simple or monotonous. Furthermore, attempts have been made to prevent the permeation of flavors through plastic containers by improving the plastic containers, but these attempts have not been widely commercialized because of the technical difficulties, the high cost of the containers, and insufficient prevention effects. For example, although hydrophilic resins such as polyvinyl alcohol or the copolymers thereof are known as plastics having a low oxygen permeability (i.e., flavor permeability), these resins cannot be used as a mono layer because they are deteriorated by water. Accordingly, although multilayer blow tubes are manufactured from hydrophilic resins laminated with polyethylene on both surfaces thereof, pumping containers manufactured from these hydrophilic resins cannot be practically utilized because of an extreme increase in costs.

Accordingly, the present invention seeks to eliminate the above-mentioned disadvantages of the prior art and to provide an oral composition capable of substantially eliminating the astringent and chalky tastes caused by formulating silicate into an oral composition, and further capable of giving the desired flavoring to the oral composition, whereby an oral composition having a good feeling upon application is provided.

The oral composition is also capable of stably maintaining the desired flavor even when stored in a plastic container for a long time by effectively preventing the escape of flavor by permeation through the plastic container.

In accordance with the present invention, there is provided an aqueous oral composition comprising (i) 0.1% to 90% by weight, based on the total amount of the composition, of a silicate and (ii) 0.01% to 10% by weight based on the total amount of the composition, of a higher aliphatic alcohol having 16 to 28 carbon atoms in a liquid state at room temperature.

The present inventors have carried intensive studies and as a result, found that, when a $C_{16}$-$C_{28}$ higher aliphatic alcohol in the form of a liquid at an ambient temperature (20°C) is formulated into an oral composition containing a silicate, the particular astringent and chalky tastes caused by the silicate are substantially eliminated and, since these higher aliphatic alcohols are tasteless and odorless, the free development of a flavor system can be effected without adversely affecting the formulation of the higher aliphatic alcohols into oral compositions. The present inventors also found that the flavor of the oral composition, for example, a mint flavor, which is normally readily permeable through a plastic container, can be substantially imprisoned therein, and thus the flavor can be stably maintained even when the oral composition contained in a container made of a plastic material having an oxygen permeability constant of 1 ml/100 inch$^2 \cdot$24 hr$\cdot$atm or more, measured at room temperature with a film thickness of 1 mil, is stored at an elevated temperature for a long time.

In comparison JP-A-59-101418 (Kokai), teaches that higher aliphatic alcohols in a solid state at an ambient temperature are formulated into oral compositions. However these alcohols neither eliminate the astringent and chalky tastes caused by silicates nor suppress the permeation of flavors through plastic containers. In addition, since these solid alcohols have particular foreign flavors, these flavors must be further masked. Conversely, the above-mentioned liquid $C_{16}$-$C_{28}$ aliphatic alcohols can unexpectedly eliminate both the astringent and chalky tastes caused by silicates and the permeability of flavors through plastic containers. Furthermore, when the liquid $C_{16}$-$C_{28}$ aliphatic alcohols are formulated into oral compositions, the alcohols are not easily hydrolyzed, and are stable and do not adversely affect the other ingredients including fluorides and other effective components in the oral compositions.

Again in comparison to the present invention Japanese Patent Application No. 59-276632 (i.e., JP-A-61-151114) does not disclose the suppression of the astringent and chalky tastes caused by silicates and the prevention of the permeation of flavors through plastic containers.

The oral composition according to the present invention may be prepared and applied as dentifrices such as a toothpaste, wet dentifrice, liquid dentifrice, mouth wash, and creamy oral agents.

Examples of the $C_{16}$-$C_{28}$ aliphatic alcohols in the liquid state at an ambient temperature (20°C) usable in the present invention are oleyl alcohol, eicosenol, docosenol, octadesenol, tetracosenol, 2-hexyldecanol, 2-octyldodecanol, 5,7,7-trimethyl-2-(1,3,3-trimethylbutyl)-1-octanol, and any mixture thereof. The so-called "Jojoba alcohol", which is a mixture of the above-mentioned alcohols, is suitable for use, to achive the purpose of the present invention. Typical compositions of the Jojoba alcohol are as follows.

| Carbon number | Name | (%) |
|---|---|---|
| 18 | Octadesenol | 6 |
| 20 | Eicosenol | 62 |
| 22 | Docosenol | 28 |
| 24 | Tetracosenol | 4 |

Of the above-mentioned liquid $C_{16}$-$C_{28}$ aliphatic alcohols, those having at least one unsaturated bond and/or these having a branched structure are preferably used in the present invention. The above-mentioned liquid $C_{16}$-$C_{28}$ aliphatic alcohols may be used alone or in any mixture thereof.

The amount of the above-mentioned liquid $C_{16}$-$C_{28}$ aliphatic alcohols, used in the composition is 0.01% to 10% by weight, based on the total weight of the oral composition. When the liquid $C_{16}$-$C_{28}$ aliphatic alcohol is used for suppressing the astringent and chalky tastes caused by silicates, the preferable formulating amount is 0.05% to 3% by weight. Conversely, when the liquid $C_{16}$-$C_{28}$ aliphatic alcohol is used for preventing the permeation of flavors through a plastic container, the preferable formulating amount is 0.1% to 10% by weight, more preferably 0.5% to 5% by weight. The use of too small an amount of the liquid $C_{16}$-$C_{28}$ aliphatic alcohol tends to result in insufficient effects, and the purposes of the present invention are not achieved. On the other hand, the use of too large an amount of the liquid $C_{16}$-$C_{28}$ aliphatic alcohol tends to produce no substantial increase of the desired effects or a suppression or the flavoring and foam generation, and to give an oily feeling.

According to the present invention, the astringent and chalky tastes caused by the presence of silicates as an abrasive, a brightening agent, or a binder in the oral composition such as toothpaste and wet dentifrice can be substantially eliminated. Typical examples of such silicates are silicate abrasives such as various synthetic amorphous silica, e.g., Zeo 49 (Registered Trade Mark) and Zeodent 113 (Registered Trade Mark, available from Huber Corp. U.S.A.), Syloid AL-1, 63, 74, and 244 (Registered Trade Mark, available from W.R.Grace, U.S.A.), Neosyl ET (Registered Trade Mark, available from J. Crossfield & Sons, England), Sident 12, 12DS, and 3 (Registered Trade Mark, available from Degussa, West Germany), HSG 756, 755, and 750 (Registered Trade Mark, available from Grace, U.S.A.), and silicates having a multivalent metal such as Al, Sr, Mg, and Ca, bonded thereto (e.g., alumino silicate, zircono silicate, and calcium silicate); and silicates, as a brightening or binder, fumed ultra finely divided silica such as Cab-O-Sil (Registered Trade Mark, available from Cabot, U.S.A.) and Aerosil (Registered Trade Mark, available from Degussa, West Germany) and finely divided hydrated silica having a secondary particle size of a few or several microns or less such as Tokusil (Registered Trade Mark, available from Tokuyama Soda, Japan), Vitasil (Registered Trade Mark, available from Tagi Chemical, Japan), and Nipsil (Registered Trade Mark, available from Nippon Silica Industrial Co.). These silicates are preferably formulated into oral compositions in an amount of 5% to 90% by weight as an abrasive, or 0.1% to 5% by weight as a brightening agent or binder, based on the total amount of the oral composition.

The permeation of flavors or perfumes in oral compositions through a plastic container can be effectively prevented by formulating the above-mentioned liquid $C_{16}$-$C_{28}$ aliphatic alcohols and silicate into the oral compositions. Thus, any flavor can be freely used regardless of the permeability constant thereof through plastic containers. For example flavors having a relatively low volatility (i.e., those which relatively do not easily permeate through plastic containers) such as accent-type flavors (e.g., wintergreen type and Cassia type) (Note: the prevention of the permeation of these flavors also can be ensured according to the present invention) and those having a large permeability through plastic containers and susceptible to a change in the flavoring (e.g., mint-type flavors such as peppermint and spearmint) can be used. Furthermore, ℓ-menthol, carvone, eugenol, and anethol may be used. Thus, the permeation of these flavors through plastic containers can be effectively prevented. These flavors are preferably formulated into oral compositions in an amount of 0.01% to 10% by weight, more preferably 0.1% to 3% by weight, based on the weight of the oral composition.

The oral compositions according to the present invention may optionally contain, in addition to the above-mentioned essential constituents, any conventional ingredients depending upon, for example, the kind of oral composition and the use thereof.

For example, in the case of dentifrices, abrasives, in addition to the above-mentioned silicates, such as calcium phosphate (dibasic), calcium carbonate, calcium pyrophosphate, insoluble sodium metaphosphate, aluminum oxide, aluminum hydroxide, and resins and further binders, in addition to the above-mentioned silicates, such as sodium carboxymethyl cellulose, hydroxyethyl cellulose, alginate, carageenan, gum arabic, and polyvinyl alcohol, can be formulated. The other optional ingredients are humectants such as polyethylene glycol, sorbitol, glycerol, and propylene glycol; surfactants such as sodium lauryl sulfate, sodium dodecylbenzene sulfonate, sodium hydrogenated coconut fatty acid monoglyceride monosulfate, sodium laurylsulfo acetate, sodium lauroyl sarcosinate, N-acyl glutamate, lauryl diethanolamide, and sucrose fatty acid ester; sweeteners such as saccharin sodium, stebiocide, neohesperidyl dihydrochalcon, glycyrrhizn, perillartine, and p-methoxy cinnamic aldehyde; preservatives; and coloring agents. In addition to the above-mentioned components, an effective component such as chlorohexidines, dextranase, mutanase, sorbin acid, alexidine, hinokitiol, cetyl pyridinium chloride, alkylglycines, alkyldiaminoethyl glycine salts, allantoin, ε-aminocaproic acid, tranexamic acid, azulene, vitamin E, water-soluble monobasic or dibasic phosphates, quaternary ammonium compounds and sodium chloride may be formulated into the present oral composition. Still other components such as sodium fluoride, sodium monofluorophosphate, and stannous fluoride may be formulated into the present oral compositions without adverse affect from the formulation of the above-mentioned liquid $C_{16}$-$C_{28}$ aliphatic alcohols.

The oral compositions according to the present invention may be prepared in any conventional manner, for example, by mixing the above-mentioned essential and optional ingredients with water.

The oral compositions containing a flavor may be advantageously filled in plastic containers, and the flavor of the oral compositions will not escape therefrom even when the oral compositions are filled in containers made of plastic materials having a relatively large oxygen permeability constant (e.g., polyolefins such as polyethylene and polypropylene and rubbers). Examples of the plastic materials usable in the present invention and the oxygen permeability constants thereof are as follows:

| Plastic material | Oxygen permeability constant* $(ml/100\ inch^2 \cdot 24\ hr \cdot atm)$ |
|---|---|
| Polypropylene | 120 - 200 |
| High-density polyethylene | 120 - 200 |
| Low-density polyethylene | 300 - 500 |
| Polystyrene | 400 - 450 |
| Polymethylmethacrylate | 7 |
| Polyvinyl acetate | 56 - 60 |
| Polyethylene terephthalate | 3.5 - 5 |
| Polyvinyl alcohol (Poval) | < 0.01 |
| Cellophane | 0.13 |
| Polyvinylidene chloride | 0.05 - 1 |
| 6,6-Nylon | 3.0 |
| 6,10-Nylon | 5 - 5.5 |
| Ethylene-vinyl acetate copolymer | > 1500 |
| Ethylene-vinyl alcohol copolymer (Eval) | < 0.005 |
| Vinyl chloride-vinyl acetate copolymer | > 20 |
| Butyl rubber | > 1500 |
| Butadiene rubber | 3000 - 4000 |
| Silicone rubber | 13000 - 15000 |
| Natural rubber | 4500 - 5000 |

\* The oxygen permeability constant was measured at room temperature, using a film having a thickness of 1 mil.

Of the above-mentioned plastic materials, those having an oxygen permeability constant of 1 or more will normally allow flavors to permeate therethrough. However, according to the present invention, such plastic materials having an oxygen permeability of 1 or more may be effectively and advantageously used.

The containers may be made by a single layer of the above-mentioned plastic material or a plurality of layers of one or more of plastic materials. The shapes of the containers are not limited. For example, blow tubes and pump containers may be used. More specifically, plastic blow tubes may be formed from, for example, polyethylene and polypropylene, and have a wall thickness of generally 300 to 400 $\mu$m. On the other hand, pump containers may be formed from a polypropylene body having a thickness of 1.0 to 1.5 mm, a polyethylene inside base having a thickness of 0.5 to 1.5 mm, and a head made of polypropylene and polyethylene in the case of the piston type and made of polyethylene, ethylenevinyl acetate copolymer, polyethylene terephthalate, or other elastomers when the head is delivered after the compression deformation (thickness = 0.5 - 1.5 mm).

As explained above and also as shown in the Examples below, according to the present invention, the astringent and chalky tastes caused by silicates can be effectively eliminated and the feeling upon application of the oral compositions can be improved by formulating the liquid $C_{16}$-$C_{28}$ higher aliphatic

alcohols into the oral compositions. In addition, since these higher aliphatic alcohols are tasteless and odorless, a free development of flavor system becomes possible, and any desired flavor can be formulated.

Furthermore the permeation of flavors in oral compositions contained in plastic containers through the plastic container can be advantageously prevented by formulating the above-mentioned liquid $C_{16}$-$C_{28}$ higheraliphatic alcohols together with the silicates into the oral compositions. Thus, the flavors of the oral compositions can be stably maintained for a long time, and any desired flavoring can be effected since a wide variety of flavors may be used.

EXAMPLES

The present invention will now be further illustrated by the following Examples and Comparative Examples, wherein "%" means "% by weight" unless otherwise specified.

Examples 1 to 3 and Comparative Examples 1 and 2

Toothpaste compositions having the formulations listed in Table 1 were prepared and the feeling upon application, i.e., astringent or chalky taste, was organoleptically evaluated by a panel of 30 experts. The evaluation was carried out by determining whether or not the panel members felt the astringent or chalky taste when actually brushing the teeth with the sample toothpaste composition. The feeling upon application of the sample toothpaste compositions was judged by the following criteria, based on the number of members who felt the astringent and chalky tastes.

Evaluation Criteria

o     Less than 5 members felt an astringent or chalky taste
Δ     5 to 20 members of 30 felt an astringent or chalky taste
x     More than 20 members felt an astringent or chalky taste
The results are shown in Table 1.

## Table 1

| Ingredient | Example | | | Comparative Example | |
|---|---|---|---|---|---|
| | 1 | 2 | 3 | 1 | 2 |
| Abrasive silica (Zeodent 113 (R)*) | 25.0% | 25.0% | – | 25.0% | – |
| Alumina hydrate | – | – | 40.0% | – | 40.0% |
| Thickening silica (Aerosil 200*) | – | – | 3.0 | – | 3.0 |
| Sorbitol | 35.0 | 35.0 | 30.0 | 35.0 | 30.0 |
| Polyethylene glycol | 5.0 | 5.0 | – | 5.0 | – |
| Sodium lauryl sulfate | 1.5 | 1.5 | 1.5 | 1.5 | 1.5 |
| Lauryl diethanolamide | – | – | 1.0 | – | 1.0 |
| Gelatin | – | – | 0.5 | – | 0.5 |
| Carageenan | – | – | 0.5 | – | 0.5 |
| Sodium alginate | – | – | 0.5 | – | 0.5 |
| Sodium carboxymethyl cellulose | 1.2 | 1.2 | – | 1.2 | – |
| Sodium saccharin | 0.1 | 0.1 | 0.1 | 0.1 | 0.1 |
| Flavor | 0.8 | 0.8 | 0.4 | 0.8 | 0.4 |

*Registered Trade Mark

EP 0 255 210 B1

Table 1 (Continued)

| Ingredient | Example | | | Comparative Example | |
|---|---|---|---|---|---|
| | 1 | 2 | 3 | 1 | 2 |
| Jojoba alcohol | 0.5 | - | 0.5 | - | - |
| 2-Octyl dodecanol | - | 0.5 | - | - | - |
| Dextranase | - | - | 2000 u/g | - | 2000 u/g |
| Sodium monofluorophosphate | 0.76 | 0.76 | - | 0.76 | - |
| Water | Balance | Balance | Balance | Balance | Balance |
| Total | 100% | 100% | 100% | 100% | 100% |
| Astringent taste | o | o | o | x | x |
| Chalky taste | o | o | o | x | △ |

As is clear from the results shown in Table 1, the higher aliphatic alcohols having 16 to 28 carbon atom suppress the astringent and chalky tastes caused by the silicates, to improve the feeling upon application.

8

EP 0 255 210 B1

| Example 4: Toothpaste | |
|---|---|
| Ingredient | % |
| Abrasive silica (Zircono silicate available from Tagi Kagaku Co.) | 25.0 |
| Polyethylene glycol 400 | 3.0 |
| Sorbitol | 40.0 |
| Sodium lauryl sulfate | 1.2 |
| Lauryl diethanolamide | 0.5 |
| Sodium carboxymethyl cellulose | 1.2 |
| Sodium saccharin | 0.2 |
| Flavor | 0.9 |
| Paraben | 0.03 |
| Thickening silica (Aerosil* available from Deggussa Co.) | 2.5 |
| Sodium monofluorophosphate | 0.9 |
| Oleyl alcohol | 0.8 |
| Water | balance |
| Total | 100.0% |

*Registered Trade Mark

| Example 5: Toothpaste | |
|---|---|
| Ingredient | % |
| Abrasive silica (Zircono silicate) | 20.0 |
| Propylene glycol | 4.0 |
| Sorbitol | 25.0 |
| Glycerol | 15.0 |
| Sodium lauryl sulfate | 0.8 |
| Sodium carboxymethyl cellulose | 1.5 |
| Sodium saccharin | 0.15 |
| Flavor | 1.5 |
| Gelatin | 1.0 |
| Paraben | 0.01 |
| Stannous fluoride | 0.82 |
| Jojoba alcohol | 0.6 |
| 2-Octyl dodecanol | 1.4 |
| Water | balance |
| Total | 100.0% |

| Example 6: Toothpaste | |
|---|---|
| Ingredient | % |
| Dicalcium phosphate | 45.0 |
| Propylene glycol | 2.5 |
| Sorbitol | 10.0 |
| Glycerol | 10.0 |
| Polyethylene glycol 400 | 2.0 |
| Sodium lauryl sulfate | 1.5 |
| Lauryl diethanolamide | 0.3 |
| Sodium carboxymethyl cellulose | 0.9 |
| Carageenan | 0.5 |
| Sodium saccharin | 0.12 |
| Flavor | 1.3 |
| Paraben | 0.02 |
| Thickening silica (Tokusil* available from Tokuyama Soda Co.) | 3.0 |
| Tranexamic acid | 0.1 |
| Jojoba alcohol | 0.3 |
| Oleyl alcohol | 0.1 |
| 5,7,7-Trimethyl-2(1,3,3-trimethylbutyl)-1-octanol | 0.3 |
| Water | balance |
| Total | 100.0% |

*Registered Trade Mark

## Example 7:   Toothpaste

| Ingredient | % |
|---|---|
| Alumina hydrate | 40.0 |
| Thickening silica (Aerosil*) | 3.0 |
| Propylene glycol | 5.0 |
| Glycerol | 25.0 |
| Sodium lauryl sulfate | 1.0 |
| Lauryl diethanolamide | 1.3 |
| Sodium alginate | 1.0 |
| Stannous fluoride | 0.41 |
| Gelatin | 0.3 |
| Sodium saccharin | 0.1 |
| Flavor | 0.9 |
| Dextranase | 500 U/g toothpaste |
| Tetracosenol | 0.5 |
| Decosenol | 2.5 |
| Water | balance |
| Total | 100.0% |

*Registered Trade Mark

# EP 0 255 210 B1

| Example 8: Toothpaste | |
|---|---|
| Ingredient | % |
| Calcium carbonate | 50.0 |
| Thickening silica (Tokusil*) | 3.0 |
| Propylene glycol | 3.0 |
| Sorbitol | 25.0 |
| Sodium lauryl sulfate | 2.3 |
| Sodium carboxymethyl cellulose | 1.5 |
| Chlorohexidine hydrochloride | 0.01 |
| Tranexamic acid | 0.2 |
| Sodium chloride | 3.0 |
| Sodium saccharin | 0.2 |
| Flavor | 1.5 |
| Sodium benzoate | 0.3 |
| Eicosenol | 0.6 |
| Docosenol | 0.2 |
| Water | balance |
| Total | 100.0% |

*Registered Trade Mark

| Example 9: Wet dentifrice | |
|---|---|
| Ingredient | % |
| Abrasive silica (Zeodent 113*) | 75.0 |
| Glycerol | 18.0 |
| Sodium carboxymethyl cellulose | 0.2 |
| Sodium lauryl sulfate | 0.5 |
| Sodium saccharin | 0.05 |
| Flavor | 0.8 |
| Sodium fluoride | 0.1 |
| 2-Hexyldecanol | 0.8 |
| Water | balance |
| Total | 100.0% |

*Registered Trade Mark

The dentifrices of Examples 4 to 9 had neither an astringent nor a chalky taste and has a good feeling upon application.

## Examples 10 to 14 and Comparative Examples 3 and 4

Toothpaste compositions having the formulations listed in Table 2 and containing a spearmint flavor, which flavor is relatively susceptible to change, were prepared. The resultant compositions were filled in pumping containers (made of polypropylene, thickness of body = 1.3 mm) or blow tubes (made of polyethylene, thickness = 300 $\mu$m).

After the toothpaste compositions were stored at a temperature of 40°C for one month, the stability of the flavor was evaluated under the following criteria, when compared with the standard flavor (i.e., immediately after preparation).

Evaluation criteria

Point 5    No substantial difference from the standard
Point 4    Slight difference from the standard

11

Point 3     Relative difference from the standard
Point 2     Substantial difference from the standard
Point 1     Big difference from the standard
The results are shown in Table 2.

Table 2

| | Example | | | | | Comparative Example | |
|---|---|---|---|---|---|---|---|
| | 10 | 11 | 12 | 13 | 14 | 3 | 4 |
| Container | Pump | Pump | Pump | Pump | Tube | Pump | Tube |
| Abrasive silica | 25.0% | 25.0% | 25.0% | 25.0% | 25.0% | 25.0% | 25.0% |
| Carboxymethyl cellulose | 1.0 | 1.0 | 1.0 | 1.0 | 1.0 | 1.0 | 1.0 |
| Glycerol | 20.0 | 20.0 | 20.0 | 20.0 | 20.0 | 20.0 | 20.0 |
| Sorbitol | 15.0 | 15.0 | 15.0 | 15.0 | 15.0 | 15.0 | 15.0 |
| Sodium saccharin | 0.15 | 0.15 | 0.15 | 0.15 | 0.15 | 0.15 | 0.15 |
| Sodium lauryl sulfate | 1.5 | 1.5 | 1.5 | 1.5 | 1.5 | 1.5 | 1.5 |
| Spearmint flavor | 1.0 | 1.0 | 1.0 | 1.0 | 1.0 | 1.0 | 1.0 |
| Jojoba alcohol | 2.0 | – | – | – | 2.0 | – | – |
| Oleyl alcohol | – | 2.0 | – | – | – | – | – |
| 2-Octyl dodecanol | – | – | 2.0 | – | – | – | – |
| 5,7,7-Trimethyl-2-(1,3,3-trimethylbutyl)-1-octanol | – | – | – | 2.0 | – | – | – |
| Water | balance | balance | balance | balance | balance | balance | balance |
| Total | 100.0% | 100.0% | 100.0% | 100.0% | 100.0% | 100.0% | 100.0% |
| Stability of flavor | 4 | 4 | 4 | 4 | 4 | 1 | 1 |

As is clear from the results shown in Table 2, in the case of the toothpaste compositions not containing $C_{16}$-$C_{28}$ higher aliphatic alcohol in the liquid state at an ambient temperature, the spearmint flavor is permeated through the plastic containers during storage at a temperature of 40°C for one month, and as a result, the flavoring of the toothpaste compositions are remarkably reduced. On the other hand, the permeation of the spearmint flavor through the plastic containers can be effectively suppressed and the flavoring can be advantageously retained in the compositions by formulating the higher aliphatic alcohols

into the compositions.

## Example 15:  Toothpaste

| Ingredient | % |
|---|---|
| Abrasive silica | 20.0 |
| Sodium carboxymethyl cellulose | 1.2 |
| Propylene glycol | 3.0 |
| Glycerol | 20.0 |
| Sodium saccharin | 0.1 |
| Sodium lauryl sulfate | 1.2 |
| Chlorohexidine hydrochloride | 0.005 |
| Paraben | 0.05 |
| Peppermint flavor | 0.9 |
| Oleyl alcohol | 5.0 |
| Water | balance |
| Total | 100.0% |

## Example 16:  Toothpaste

| Ingredient | % |
|---|---|
| Zircono silicate | 25.0 |
| Sodium carboxymethyl cellulose | 1.0 |
| Polyethylene glycol 400 | 4.0 |
| Sorbitol | 30.0 |
| Sodium saccharin | 0.1 |

13

| Sodium lauryl sulfate | 1.2 |
| Sodium monofluorophosphate | 0.76 |
| Paraben | 0.02 |
| Spearmint flavor | 1.0 |
| Jojoba alcohol | 3.0 |
| Water | balance |
| Total | 100.0% |

## Example 17: Toothpaste

| Ingredient | % |
| --- | --- |
| Alumino silicate | 20.0 |
| Sodium carboxymethyl cellulose | 1.0 |
| Polyethylene glycol 400 | 3.0 |
| Sorbitol | 25.0 |
| Titanium dioxide | 1.0 |
| Sodium saccharin | 0.12 |
| Sodium lauryl sulfate | 1.5 |
| Paraben | 0.01 |
| Wintergreen flavor | 1.0 |
| Eicosenol | 1.0 |
| Jojoba alcohol | 1.0 |
| Water | balance |
| Total | 100.0% |

## Example 18: Toothpaste

| Ingredient | % |
| --- | --- |
| Abrasive silica | 30.0 |
| Sodium carboxymethyl cellulose | 1.1 |
| Propylene glycol | 3.0 |
| Sorbitol | 25.0 |
| Sodium saccharin | 0.1 |
| Sodium lauryl sulfate | 1.3 |
| Lauryl diethanolamide | 0.4 |
| Sodium monofluorophosphate | 0.8 |
| Titanium dioxide | 0.5 |

| | |
|---|---|
| Spearmint flavor | 0.9 |
| 5,7,7-Trimethyl-2-(1,3,3-tri-methylbutyl)-1-octanol | 2.0 |
| Water | balance |
| **Total** | **100.0%** |

The toothpaste compositions of Examples 15 to 18 were filled in polypropylene pumping containers having a body thickness of 1.3 mm, and the toothpaste compositions of Examples 16 to 18 were filled in polyethylene blow tubes having a thickness of 300 $\mu$m.

When the above-mentioned toothpaste compositions were stored at a temperature of 40°C for one month, the flavor retention was good.

| Example 19: Toothpaste | |
|---|---|
| Ingredient | % by weight |
| Abrasive silica | 25 |
| Sodium alginate | 0.3 |
| Hydroxyethyl cellulose | 1.0 |
| Gelatin | 0.3 |
| Polyethylene glycol | 5.0 |
| Sorbitol liquid | 60 |
| Sodium lauryl sulfate | 1.0 |
| Myristyl diethanolamide | 1.0 |
| Sodium saccharin | 0.1 |
| Dextranase | 2000 U/g toothpaste |
| Carvacrol | 0.03 |
| Sodium fluoride | 0.2 |
| Flavor | 1.0 |
| Jojoba alcohol | 0.8 |
| Paraben | 0.01 |
| Coloring agent | q.s. |
| Purified water | balance |
| Total | 100.0% |

| Example 20: Toothpaste | |
|---|---|
| Ingredient | % |
| Aluminum hydroxide | 45.0 |
| Thickening silica | 2.0 |
| Sorbitol Solution (60%) | 30.0 |
| Propylene glycol | 3.0 |
| Carageenan | 0.5 |
| Sodium alginate | 0.5 |
| Sodium lauryl sulfate | 1.0 |
| Sodium saccharin | 0.1 |
| Gelatin | 0.5 |
| Flavor | 1.0 |
| Myristyl diethanolamide | 1.5 |
| Dextranase | 2000 U/g toothpaste |
| Jojoba alcohol | 0.3 |
| Isopropylmethylphenol | 0.03 |
| Purified water | balance |
| Total | 100.0% |

## Claims

1. An aqueous oral composition comprising (i) 0.1% to 90% by weight, based on the total amount of the composition, of a silicate and (ii) 0.01% to 10% by weight based on the total amount of the composition, of a higher aliphatic alcohol having 16 to 28 carbon atoms in a liquid state at room temperature.

2. An oral composition as claimed in claim 1, wherein said higher aliphatic alcohol has a branched structure.

3. An oral composition as claimed in claim 1, wherein said higher aliphatic alcohol has at least one unsaturated bond.

4. An oral composition as claimed in claim 1, wherein said higher aliphatic alcohol is Jojoba alcohol.

## Patentansprüche

1. Wäßrige orale Zusammensetzung, umfassend (i) 0,1 Gew.-% bis 90 Gew.-%, bezogen auf die Gesamtmenge der Zusammensetzung, eines Silikats und (ii) 0,01 Gew.-% bis 10 Gew.-%, bezogen auf die Gesamtmenge der Zusammensetzung, eines bei Raumtemperatur flüssigen höheren aliphatischen Alkohols mit 16 bis 28 Kohlenstoffatomen.

2. Orale Zusammensetzung nach Anspruch 1, worin der höhere aliphatische Alkohol eine verzweigte Struktur aufweist.

3. Orale Zusammensetzung nach Anspruch 1, worin der höhere aliphatische Alkohol mindestens eine ungesättigte Bindung aufweist.

4. Orale Zusammensetzung nach Anspruch 1, worin der höhere aliphatische Alkohol Jojoba-Alkohol ist.

## Revendications

1. Composition buccale aqueuse comprenant (i) 0,1 % à 90 % en poids, basé sur la quantité totale de la composition, d'un silicate et (ii) 0,01 % à 10 % en poids basé sur la quantité totale de la composition, d'un alcool aliphatique supérieur ayant 16 à 28 atomes de carbone dans un état liquide à température ambiante.

2. Composition buccale selon la revendication 1, dans laquelle le dit alcool aliphatique supérieur a une structure branchée.

3. Composition buccale selon la revendication 1, dans laquelle le dit alcool aliphatique supérieur a au moins une liaison insaturée.

4. Composition buccale selon la revendication 1, dans laquelle le dit alcool aliphatique supérieur est l'alcool Jojoba.